# EUROPEAN PATENT SPECIFICATION

(11) **EP 3 811 948 B1**
(45) Date of publication and mention of the grant of the patent: **11.09.2024**
(21) Application number: 19822580.7
(22) Date of filing: 21.06.2019
(51) Int. Cl.: A61K 31/7034, A61P 3/10, A61P 13/12, A61P 27/02, A23L 33/125

(54) **GLYCOSIDES FOR USE IN PREVENTING AND TREATING DIABETIC COMPLICATIONS**
GLYCOSIDE ZUR VORBEUGUNG UND BEHANDLUNG DIABETISCHER KOMPLIKATIONEN
GLYCOSIDES POUR LEUR UTILISATION DANS LA PRÉVENTION ET LE TRAITEMENT DES COMPLICATIONS ASSOCIÉES AU DIABÈTE

(30) Priority: 22.06.2018 CN 201810651588
(43) Date of publication of application: 28.04.2021
(73) Proprietor: Chengdu Sinnocean Biotechnology Co., Ltd., Chengdu, Sichuan 610000 (CN)
(72) Inventor: YANG, Cheng, Chengdu, Sichuan 610000 (CN); SU, Dan, Chengdu, Sichuan 610000 (CN); ZHONG, Zhihui, Chengdu, Sichuan 610000 (CN)
(74) Representative: Altmann Stößel Dick Patentanwälte PartG mbB
(86) International application number: PCT/CN2019/092383
(87) International publication number: WO 2019/242764

(56) References cited:
- CN-A- 103 232 350
- CN-A- 107 519 191
- YAHARA SHOJI ET AL: "Isolation and characterization of phenolic compounds from Coptidis Rhizoma.", CHEMICAL AND PHARMACEUTICAL BULLETIN, vol. 33, no. 2, 1 January 1985 (1985-01-01), JP, pages 527 - 531, XP055893054, ISSN: 0009-2363, DOI: 10.1248/cpb.33.527
- LI XUE-GAI; YANG LI-GUO; CHEN LI-XIA; QIU FENG: "Chemical constituents from the decoction of Coptis chinensis Franch", JOURNAL OF SHENYANG PHARMACEUTICAL UNIVERSITY, vol. 29, no. 3, 1 March 2012 (2012-03-01), pages 193 - 198, XP009533695, ISSN: 1006-2858
- ZHANG ZENG-GUANG ET AL: "New phenolic glycosides from Anemone chinensis Bunge and their antioxidant activity", NATURAL PRODUCT RESEARCH, 28 April 2021 (2021-04-28), GB, pages 1 - 7, XP055893057, ISSN: 1478-6419, DOI: 10.1080/14786419.2021.1917569
- ZHANG FENG-XIANG ET AL: "Dissection of the potential pharmacological mechanism of Rhizoma coptidis water extract against inflammation in diabetes mellitus via chemical profiling, network pharmacology and experimental validation", NEW JOURNAL OF CHEMISTRY, vol. 45, no. 35, 13 September 2021 (2021-09-13), GB, pages 16283 - 16297, XP055893053, ISSN: 1144-0546, DOI: 10.1039/D1NJ02812J
- CHEN, YING ET AL.: "Evaluating Pharmacological Effects of Two Major Components of Shuangdan Oral Liquid: Role of Danshensu and Paeonol in Diabetic Nephropathy Rat", BIOMOLECULES & THERAPEUTICS, vol. 24, no. 5, 1 September 2016 (2016-09-01), pages 536 - 542, XP053036949, ISSN: 1976-9148

## Description

### Technical field

The present invention relates to the field of medicine, in particular to a glycoside compound for use in the prevention and the treatment of a diabetic complication, namely a microvascular disease.

### Background art

As the development of society, the prevalence of diabetes (Diabetes Mellitus, DM) has significantly increased, and DM has become one of the important diseases affecting human health. Diabetes is a common endocrine and metabolic disease with metabolic disorders of protein and fat as the main clinical manifestation caused by the relative or absolute lack of insulin secretion in the body. According to the 8th edition of the Global Diabetes Map released by the International Diabetes Federation (IDF), there were 425 million diabetic patients worldwide in 2017, and it is estimated that there will be nearly 700 million diabetic patients in 2045. The symptomatic treatment of diabetes is to control the blood sugar index, but the patient needs to take medicine for life, and as the progress of this disease, complications such as hypertension, coronary heart disease, hyperlipidemia, retinopathy, diabetic nephropathy and sick feet and the similar will occur. According to the statistics from the World Health Organization, more than 50% of diabetic deaths are caused by cardiovascular and cerebrovascular diseases, and 10% are caused by nephropathy. Clinical data show that about 10 years after the onset of diabetes, 30% to 40% of patients will have at least one complication, and once the complication is developed, it is difficult to reverse it with medical treatment, so diabetic complications should be prevented as soon as possible.

Diabetic microangiopathy is more specific, and its main characteristics are thickened basement membrane and deposition of transparent substances. The patients with diabetes have various degrees of abnormality in the microcirculation, and the abnormality caused by basement membrane disease and that caused by microcirculation affect each other, which promotes the progression and development of microvascular disease. Microangiopathy mainly manifests in the retina, kidney, myocardium, nerve tissue and toes. Clinically, diabetic retinopathy, diabetic nephropathy and diabetic neuropathy are often used as the main places to reflect diabetic microangiopathy.

Diabetic retinopathy (DR) is one of the most common microvascular complications of diabetes, and it is the main cause of vision loss in the elderly. The longer the course of diabetes, the worse the blood sugar control, the higher the risk of retinopathy and impaired vision. When the retinal blood vessel wall begins to be destroyed, it means that retinopathy is developed. When the abnormal blood vessel wall oozes blood or fluid into the eyeball, it can cause vision loss or distortion. The prevalence of retinopathy in diabetic patients is relatively high, while the incidence of proliferative diseases is 3.3% to 7.4%. The pathogenesis of diabetes is not fully understood, but inflammation, oxidative stress and microvascular changes play an important role in the deterioration of retinal function. Prior methods for the treatment of diabetic retinopathy are mainly prevented by controlling blood sugar, blood lipids, blood pressure and other indicators in the early stage of diabetes, as well as treated by physical means such as laser, condensation or vitrectomy after onset of this disease. For the later drug treatment, only Lucentis (Ranibizumab injection) developed by Genentech is commercially available for the treatment of retinopathy with or without macular edema (DME), but it is expensive. Therefore, developing novel drugs for the treatment of retinopathy is a problem that we currently need to solve.

Diabetic nephropathy is one of the most serious complications of diabetes, and is also the main microvascular complication of diabetes. It mainly refers to diabetic glomerulosclerosis, a glomerular disease mainly with vascular damage. In the early stage, it is mostly asymptomatic, and blood pressure can be normal or high. Its incidence increases as the prolonged course of diabetes. In the early stage of diabetes, the kidney volume swells, and the glomerular filtration rate increases, presenting a state of high filtration. Later, interstitial proteinuria or microalbuminuria will gradually appear. With the prolongation of the disease course, persistent proteinuria, edema, hypertension, and decreased glomerula filtration rate, further renal insufficiency and uremia, are one of the main causes of death from diabetes.

Chen, Ying et al., Biomol. Therap. 2016, 24, 536, discloses that a combination of danshensu and paenol could delay the process of diabetic nephropathy in rats. CN 103 232 350 A discloses the use of danshensu derivatives in the treatment of diabetic complications such as glomerulonephritis and diabetic nephropathy.

The etiology and pathogenesis of microvascular diseases such as diabetic retinopathy and nephropathy are not yet clear, and they are generally caused by various risk factors and are irreversible. Blood sugar can be effectively controlled, but complications cannot be correspondingly controlled.

### Content of the invention

The technical problem to be solved by the present invention is that prior methods for treatment of diabetic microvascular disease are mainly by preventing the occurrence of the disease via controlling blood glucose, blood lipid, blood pressure and other indicators, as well as treating via physical means such as laser, condensation or vitrectomy after the occurrence of the disease. However, the drugs for the treatment of diabetic microangiopathy are rare, but the present invention provides the use of glycoside compounds in the preparation of drugs for preventing and treating diabetic complications, to solve the above problems, that is especially beneficial to the treatment of diabetic microangiopathy. The present invention is realized through the following technical solutions:
The present invention provides a compound represented by formula (IV), or a pharmaceutically acceptable salt, or a solvate thereof for use in the prophylaxis and the treatment of a diabetic complication: 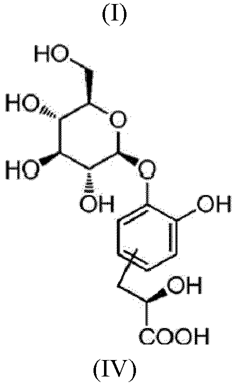 wherein the complication is a microvascular disease.

Further, the complications arc caused by type 1 diabetes or type 2 diabetes.

Further, the microvascular disease is nephropathy and retinopathy.

Further, the prepared drug is administered by sublingual administration, inhalation, oral administration or injection. Among them, injection includes intradermal injection, subcutaneous injection, intramuscular injection, and intravenous injection.

A pharmaceutical composition is disclosed for use as a drug for preventing and treating diabetic complications, that is obtained by using the compound mentioned above, or a pharmaceutically acceptable salt, or a solvate thereof as an active ingredient, with the addition of pharmaceutically acceptable excipients.

In the present invention, "pharmaceutically acceptable" means that certain carrier, vehicle, diluent, excipient, and/or formed salt is usually chemically or physically compatible with other ingredients constituting a certain pharmaceutical dosage form, as well as physiologically compatible with the acceptor.

In the present invention, "salt" means acid and/or basic salt that is formed by reaction of a compound or its stereoisomer with inorganic and/or organic acid and base, and also includes zwitterionic salts (inner salts), and further includes quaternary ammonium salts, such as alkylammonium salt. These salts can be directly obtained during the final isolation and purification of a compound. The salts can also be obtained by mixing a compound or its stereoisomers with a certain amount of acid or base appropriately (for example, in equivalent). These salts may form a precipitate in the solution, and be collected by filtration, or recovered after evaporation of the solvent, or obtained by freeze-drying after reaction in an aqueous medium. The salt in the present invention may be hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate.

The present invention has the following advantages and beneficial effects.

The object of the present invention is to provide a glycoside compound for use in the treatment of a diabetic complication which is a microvascular disease, wherein the compound shows an obvious action on lowering blood sugar, and has a good therapeutic effect on diabetes;
the compound can reduce the levels of UREA and CREA to a certain extent, show a tendency of lowering the proliferation of glomerular mesangial or mesangial cells, and have a certain protective effect on the kidney; at the same time, the compound can significantly reduce the degeneration degree of the retinal ganglion cell layer, indicating that the compound has an effect of reducing the degeneration of the retinal ganglion cell layer in animals. Therefore, the compound has a certain degree of protective effect on the kidney and retina of diabetic animals, and has good economic and social value. In addition, the amount of the compound used for the treatment of diabetic lesions is related to the dosage, and the more the amount of the compound, the better the effect.

### Description of figures

The figures described here are used to provide a further understanding of the embodiments of the present invention, constitute a part of the application, and do not constitute a limitation to the examples of the present invention. In the figures:
Figure 1 shows the results of lowering blood sugar in representative animals of each group according to the present invention;
Figure 2 is a pathological staining picture of the retina from representative animals of each group according the present invention;
Figure 3 is the score for the retinal ganglion cell layer of each group animals in the present invention; wherein, *P < 0.05, **P < 0.01, ***P < 0.001 vs. negative control group;
Figure 4 is a pathological staining diagram of the kidneys from representative animals in each group;
Figure 5 shows the pathological scores of kidney glomeruli in each group of animals.

### Examples

In order to make the object, technical solutions, and advantages of the present invention be more easily understood, the present invention will be further described in detail below, combined with the examples and the figures. The exemplary embodiments and descriptions of the present invention are only used to illustrate the present invention, not as a limitation of the present invention.

### Example 1 Pharmacological activity of glycoside compounds

The investigation of the therapeutic effect of H2 (i.e. compound of formula (IV)) on spontaneous type II diabetic db/db mice was tested, and the specific experiment and results are as follows:

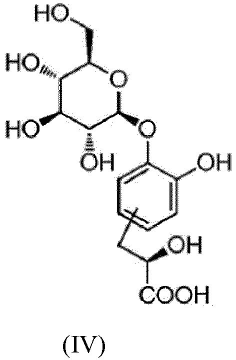

The compound can be prepared by the preparative method of compound **15** reported in the literature "Isolation and identification of chemical components of Coptis chinensis aqueous extract" (Li Xuegai et al., Journal of Shenyang Pharmaceutical University, March 2012, volume 29, issue 3, pages 193-198) 15 is obtained by the preparation method.

### 1. Regulations followed by the test:

"Provisions for Drug Registration" (China Food and Drug Administration Order No. 28, October 01, 2007);
"Good Laboratory Practice" (China Food and Drug Administration Order No. 2, September 01, 2003).

### 2. Experimental materials

### 2.1 Testing sample

Name or code: H2;
Source: Laboratory of Chengdu Zhongchuang Shuyang Biotechnology Co., Ltd.;
Properties: solid;
Storage conditions: ≤ -15 °C, airtight and dry;
Preparative method: prepared in distilled water;
Label after preparation: indicating the experimental No., the name of test substance and the concentration, the preparative volume, storage conditions, expiration date, responsible person and preparative date, etc.;
Temporary storage conditions after preparation: airtight, 2 to 8 °C;
Expiration date after preparation: 5 days;

### 2.2 Reference substance

Name or code: Metformin;
Source: Sine-America Shanghai Squibb Pharmaceutical Co., Ltd.;
Properties: solid;
Specification and concentration: 0.5 g/tablet;
Lot number: AAN0329;
Storage conditions: 2 to 8 °C, airtight and dry;
Expiration date: November 2018;
Preparative method: prepared in distilled water;
Label after preparation: indicating the experimental No., the name of test substance and the concentration, the preparative volume, storage conditions, expiration date, responsible person and preparative date, etc.;
Temporary storage conditions after preparation: airtight, 2 to 8 °C;
Expiration date after preparation: 5 days;

### 2.3 Solvent reference substance

Name or code: distilled water;
Properties after preparation: colorless and clear liquid;
Storage conditions: 2 to 8 °C, airtight and dry;
Label after preparation: indicating the experimental No., the name of test substance and the concentration, the preparative volume, storage conditions, expiration date, responsible person and preparative date, etc.;

### 2.4 Other main reagents

50% Glucose injection (product of Hubei Kelun Pharmaceutical Co., Ltd., specification: 20 mL/bottle, batch number: B160915B).
Accu-Chek Performa test strips (product of Roche Diognostics GmbH Company, specification: 100 test strips/box, batch number: 475761).

### 2.5 Main instruments and equipment

ACCU-CHEK Performa (product of Roche Diognostics GmbH Company)_{∘}

### 3. Experimental system

### 3.1 Experimental animals

Species: db/db, db/m mice;
Level: SPF grade;
Number and sex of animals used: db/db 72 mice (male), db/m 12 mice (male);
Age: about 9 weeks old;
Body weight: the average value of db/m mice was 26.7 to 34.0 g, and the value of individual weight was within ±20% of the mean (the animal weight at the beginning of the experiment); the average value of db/db mice was 40.7 to 55.6 g, and the value of individual weight was in the range of ±20% mean (animal weight at the beginning of the experiment);
Source: Changzhou Cavins Laboratory Animal Co., Ltd.; production license number: SCXK (Su)2016-0010.

### 3.2 Feeding management

### (1) Environmental adaptation

The main detection results during the period of adaptation: consistent with the quality indicators required at the time of ordering; the general state of the animal being normal; the weight of the animal being in the weight range required by the experiment; not including abnormal unqualified animals in the experiment.

### (2) Feeding place

SPF area of WestChina-Frontier Pharmaceutical Technology Co., Ltd. (Building 3) (Experimental animal license number: SYXK (Chuan) 2009-123).

### (3) Feeding conditions

Housing density: ≤ 5/cage;
Frequency of cage space displacement: < 1 time/week.

### (4) Feeding environmental conditions

Breeding environmental condition standard: National Standard of the People's Republic of China GB14925-2010.

### (5) Feed

Kind: maintenance feed for rats and mice;
Manufacturing unit: provided by Shanghai Slack Laboratory Animal Co., Ltd.;
Feeding method: free intake (except when the experiment has special requirements);
Nutrient composition testing: conventional nutrient composition indicators: crude protein, crude fat, crude fiber, crude ash, moisture, calcium and phosphorus; amino acid indicators: threonine, cystine+methionine, valine, isoleucine, leucine, tyrosine+phenylalanine, histidine, lysine, arginine, tryptophan, referring to the national standard of the People's Republic of China GB14924.3-2010;
Confirmation of feed pollutant content: chemical pollutant indicators: arsenic, lead, cadmium, mercury, hexachlorocyclohexane, clofenotane, aflatoxin B1, total number of colonies, coliforms, mold and yeast counts, pathogenic bacteria (Salmonella), referring to the National Standard of the People's Republic of China GB14924.2-2001.

### (6) Drinking water

Type: drinking water for laboratory animal (reverse osmosis water);
Water supply method: drinking water in bottle, free access.

### 4. Experimental method

### 4.1 Model building

db/db mouse model: blood glucose of > 7.8 mmol/L was included in the type 2 diabetes model group (the minimum blood glucose in the db/db model group was 7.8 mmol/L, which was significantly higher than the average value of 5.6 in the db/m control group).

### 4.2 Animal grouping

Design of test groups: db/m group, db/db mice are divided into the following six groups: blank control group (no glucose), negative control group (model group), positive control group (350 mg/kg metformin), H2 low dose group (40 mg/kg), H2 medium dose group (80 mg/kg), H2 high dose group (160 mg/kg);
Number of animals: 12 mice for each group;
Sex: male;
Grouping method: mice were randomly divided into groups based on the fasting blood glucose obtained before the first administration, and for specific grouping information, see Table 1 below.

**Table 1 Information table for animal grouping**

| Groups | Animal number after grouping | Animal number before grouping | Animal weight (g) | Animal blood glucose (mmol/L) |
|---|---|---|---|---|
| Group db/m | 1M0101 | M2001 | 31.2 | 6.6 |
| | 1M0102 | M2105 | 32.7 | 5.9 |
| | 1M0103 | M1903 | 31.3 | 4.3 |
| | 1M0104 | M1905 | 29.8 | 5.7 |
| | 1M0201 | M2103 | 26.7 | 5.0 |
| | 1M0202 | M2104 | 31.9 | 5.9 |
| | 1M0203 | M1904 | 32.8 | 6.7 |
| | 1M0204 | M2102 | 34.0 | 4.9 |
| | 1M0301 | M2106 | 28.6 | 4.1 |
| | 1M0302 | M2004 | 31.5 | 5.5 |
| | 1M0303 | M2003 | 31.3 | 7.0 |
| | 1M0304 | M1902 | 27.3 | 6.2 |
| Blank control group | 2M0101 | M0603 | 45.9 | 14.3 |
| | 2M0102 | M1703 | 49.9 | 16.3 |
| | 2M0103 | M1205 | 47.5 | 9.7 |
| | 2M0104 | M 1604 | 40.7 | 11.0 |
| | 2M0201 | M0804 | 50.0 | 9.9 |
| | 2M0202 | M0803 | 48.0 | 9.7 |
| | 2M0203 | M0905 | 49.5 | 17.1 |
| | 2M0204 | M1002 | 47.8 | 14.4 |
| | 2M0301 | M1403 | 52.3 | 7.8 |
| | 2M0302 | M0105 | 47.7 | 23.9 |
| | 2M0303 | M0604 | 46.5 | 10.1 |
| | 2M0304 | M0103 | 47.0 | 12.2 |
| Negative control group | 3M0101 | M0302 | 51.5 | 10.0 |
| | 3M0102 | M1705 | 48.3 | 12.0 |
| | 3M0103 | M0104 | 43.8 | 21.4 |
| | 3M0104 | M0303 | 44.6 | 9.4 |
| | 3M0201 | M0304 | 51.3 | 15.0 |
| | 3M0202 | M1202 | 46.3 | 10.8 |
| | 3M0203 | M0502 | 45.9 | 16.8 |
| | 3M0204 | M0902 | 51.9 | 8.4 |
| | 3M0301 | M0305 | 41.7 | 19.1 |
| | 3M0302 | M0904 | 50.0 | 7.8 |
| | 3M0303 | M1304 | 47.4 | 10.0 |
| | 3M0304 | M0702 | 44.2 | 15.8 |
| Positive control group | 4M0101 | M1402 | 48.0 | 9.4 |
| | 4M0102 | M1302 | 49.7 | 8.8 |
| | 4M0103 | M0504 | 45.5 | 28.6 |
| | 4M0104 | M1805 | 51.8 | 11.2 |
| | 4M0201 | M1204 | 48.9 | 14.0 |
| | 4M0202 | M1605 | 47.9 | 8.8 |
| | 4M0203 | M0404 | 48.4 | 21.8 |
| | 4M0204 | M1804 | 48.1 | 10.9 |
| | 4M0301 | M0402 | 44.3 | 18.0 |
| | 4M0302 | M1504 | 44.1 | 9.4 |
| | 4M0303 | M 1603 | 48.9 | 11.4 |
| | 4M0304 | M0201 | 48.1 | 11.1 |
| H2 low dose group | 5M0101 | M0704 | 47.0 | 10.9 |
| | 5M0102 | M1704 | 47.2 | 13.9 |
| | 5M0103 | M1702 | 49.6 | 8.2 |
| | 5M0104 | M0202 | 53.6 | 27.5 |
| | 5M0201 | M1503 | 51.5 | 9.4 |
| | 5M0202 | M0405 | 50.3 | 14.5 |
| | 5M0203 | M0503 | 47.5 | 18.3 |
| | 5M0204 | M0701 | 47.1 | 7.9 |
| | 5M0301 | M0204 | 51.0 | 22.6 |
| | 5M0302 | M1404 | 54.5 | 9.9 |
| | 5M0303 | M1003 | 46.0 | 13.4 |
| | 5M0304 | M1203 | 46.4 | 9.3 |
| H2 medium dose group | 6M0101 | M1105 | 49.9 | 9.4 |
| | 6M0102 | M0401 | 49.7 | 18.0 |
| | 6M0103 | M1102 | 48.4 | 10.2 |
| | 6M0104 | M0601 | 54.2 | 10.9 |
| | 6M0201 | M1004 | 43.2 | 10.8 |
| | 6M0202 | M0205 | 45.2 | 20.0 |
| | 6M0203 | M0203 | 50.2 | 14.7 |
| | 6M0204 | M1803 | 48.6 | 8.7 |
| | 6M0301 | M0403 | 44.7 | 13.2 |
| | 6M0302 | M0505 | 44.6 | 35.0 |
| | 6M0303 | M0901 | 44.9 | 9.4 |
| | 6M0304 | M1101 | 54.1 | 17.0 |
| H2 high dose group | 7M0101 | M1005 | 54.8 | 12.5 |
| | 7M0102 | M1305 | 48.6 | 27.1 |
| | 7M0103 | M0801 | 42.7 | 8.0 |
| | 7M0104 | M0301 | 48.9 | 8.3 |
| | 7M0201 | M1502 | 45.4 | 11.2 |
| | 7M0202 | M1501 | 55.6 | 9.0 |
| | 7M0203 | M0501 | 47.1 | 26.1 |
| | 7M0204 | M0903 | 50.5 | 9.7 |
| | 7M0301 | M1505 | 47.0 | 10.0 |
| | 7M0302 | M1201 | 48.8 | 14.3 |
| | 7M0303 | M0101 | 47.2 | 7.8 |
| | 7M0304 | M0605 | 46.3 | 22.2 |

### 4.3 Dosage

Specific dosage design was list in Table 2.

**Table 2 Dosing plan**

| Groups | Route of administration | Dosage (mg/kg) | Dosing concentration (mg/mL) | Dosing volume (mL/kg) | Number of animals |
|---|---|---|---|---|---|
| | | | | | male |
| Group db/m | Oral gavage | - | / | 10 | 12 |
| Blank control group | Oral gavage | - | / | 10 | 12 |
| Negative control group | Oral gavage | - | / | 10 | 12 |
| Positive control group | Oral gavage | 350 | 35 | 10 | 12 |
| H2 low dose group | Oral gavage | 40 | 4 | 10 | 12 |
| H2 medium dose group | Oral gavage | 80 | 8 | 10 | 12 |
| H2 high dose group | Oral gavage | 160 | 16 | 10 | 12 |

Administration frequency: once a day, for 12 weeks; db/m group, blank control group and negative control group: receiving distilled water; positive control group: positive drug metformin; other administration groups: receiving different dose of test drug H2, respectively; the day of administration was defined as the first day of the test.

### 5. Statistical analysis and result assessment

The data obtained were analyzed using EXCEL and IBM SPSS Statistics22.

The homogeneity test of variance was first performed, and when the variance was uniform (P ≥ 0.05), the repeated measures analysis of variance (Repeated measures ANOVA) was used to statistically test the group difference of blood glucose and weight. When the group difference was statistically significant (P < 0.05), the LSD method in one-way ANOVA was used to compare the differences between groups at each time point; when repeated measures analysis of variance showed that the differences between groups were not statistically significant (P ≥ 0.05), the statistical analysis was over. When the variance was not uniform (P < 0.05), Kruskal-Wallis H rank-sum test (K-W method) was used for statistical analysis. When Kruskal-Wallis H rank-sum test showed statistical significance (P < 0.05), Mann-Whitney U test (M-W method) was used to compare the differences between groups at each time point; when Kruskal-Wallis H rank-sum test showed that the difference was not statistically significant (P ≥ 0.05), the statistical analysis ended. For group differences in the mean of other indicators, when the variance was uniform (P ≥ 0.05), ONE-WAY ANOVA was used to statistically test the group difference, when the group difference was statistically significant (P < 0.05), LSD method was used to compare the differences between groups; when ONE-WAY ANOVA showed that the differences between groups were not statistically significant (P ≥ 0.05), the statistical analysis was over. When the variance was not uniform (P < 0.05), Kruskal-Wallis H rank-sum test (K-W method) was used for statistical analysis. When Kruskal-Wallis H rank-sum test showed statistical significance (P < 0.05), Mann-Whitney U test (M-W method) was used to compare the differences between groups; when Kruskal-Wallis H rank-sum test showed that the difference was not statistically significant (P ≥ 0.05), the statistical analysis ended.

### 6. Experimental results

### 6.1 Hypoglycemic effect test

Sampling frequency: blood glucose was detected once every 2 weeks after grouping, and then after glucose was administered, the blood glucose level was further measured. Sampling site: tail vein; sampling volume: about 1 drop; blood glucose detection: real-time detection by blood glucose meter.

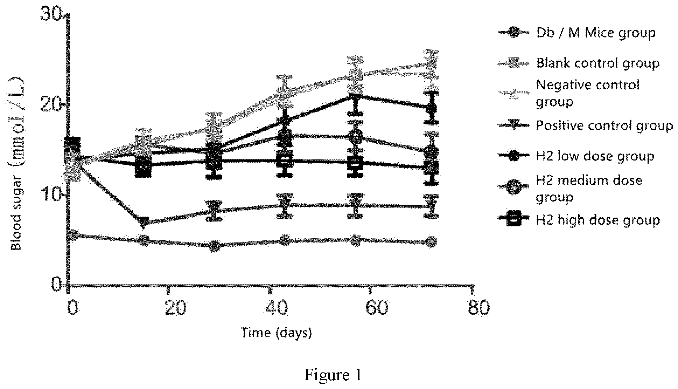

The blood glucose level of each animal group was shown in Table 3 and Figure 1. During the administration period, the blood glucose level of the mice in the blank control group and the negative control group presented an upward trend. Compared with the negative control group, the blood glucose level of the positive drug metformin group was significantly lower than that of the negative control group from the 15^{th} day after administration (P < 0.001), indicating that the positive drug could significantly reduce the blood sugar level of db/db mice; while the low-dose test drug H2 did not show obvious hypoglycemic effect. The blood glucose level of the middle-dose group was significantly lower than that of the negative control group (P < 0.01) from the 15^{th} day after administration, and the blood glucose level was significantly lower than that of the negative control group (P < 0.001) on the 72^{th} day after administration. The blood glucose level of mice in the high-dose test drug H2 group was rather significantly lower than that of the negative control group on day 43 after administration, i.e. (20.84 ± 0.66) vs (13.87 ± 1.76) mmol/L (P < 0.01), and on the 57^{th} day after administration, above-mentioned changes were more obvious, i.e. (23.46 ± 1.87) vs (13.07 ± 1.71) mmol/L (P < 0.001). The blood glucose level of mice in the db/m group did not change significantly during the whole experiment and was significantly lower than that of the other groups. It showed that the hypoglycemic effect of the positive drug and the high-dose group of the test drug was equivalent, and even better than that of the high-dose group.

**Table 3 Blood glucose concentration of each group (MEAN ± SEM, mmol/L)**

| Groups | Dosage (mg/kg) | Blood glucose concentration (mmol/L) | | | | | |
|---|---|---|---|---|---|---|---|
| | | Administrating for one day | Administrating for 15 days | Administrating for 29 days | Administrating for 43 days | Administrating for 57 days | Administrating for 72 days |
| db/m group | | 5.63 ± 0.26 *** | 5.01 ± 0.25 *** | 4.39 ± 0.17 *** | 5.00 ± 0.34 *** | 5.07 ± 0.27 *** | 4.87 ± 0.34 *** |
| Blank control group | - | 13.17±1.25 | 15.46 ± 0.86 | 17.63 ± 1.45 | 21.48 ± 1.66 | 23.45 ± 1.33 | 24.61 ± 1.37 |
| Negative control group | - | 13.33 ± 1.2 | 16.23 ± 1.13 | 17.31 ± 1.00 | 20.84 ± 0.66 | 23.46 ± 1.87 | 23. 57 ± 1.69 |
| Positive control group | 350 | 13.78 ± 1.74 | 6.95 ± 0.57 *** | 8.30 ± 0.94 *** | 8.93 ± 1.15 *** | 8.92 ± 1.12 *** | 8.80 ± 1.11 *** |
| H2 low dose group | 40 | 14.07 ± 1.71 | 14.66 ± 1.79 | 15.08 ± 1.99 | 18.31 ± 1.95 | 21.14 ± 1.97 | 19.71 ± 1.60 |
| H2 medium dose group | 80 | 13.17 ± 1.06 | 15.67 ± 1.54 | 14.69 ± 1.75 | 16.64 ± 1.75 ** | 16.54 ± 1.50 ** | 14.81 ± 1.92 *** |
| H2 high dose group | 160 | 14.3 ± 1.95 | 13.28 ± 1.06 | 13.82 ± 1.74 | 13.87 ± 1.76 ** | 13.60 ± 1.38 *** | 13.07 ± 1.71 *** |

| | | | | | | | |
|---|---|---|---|---|---|---|---|
| Note: *P<0.05, **P<0.01, ***P<0.001 vs. negative control group. | | | | | | | |

### 6.2 Prevention and treatment of retinopathy

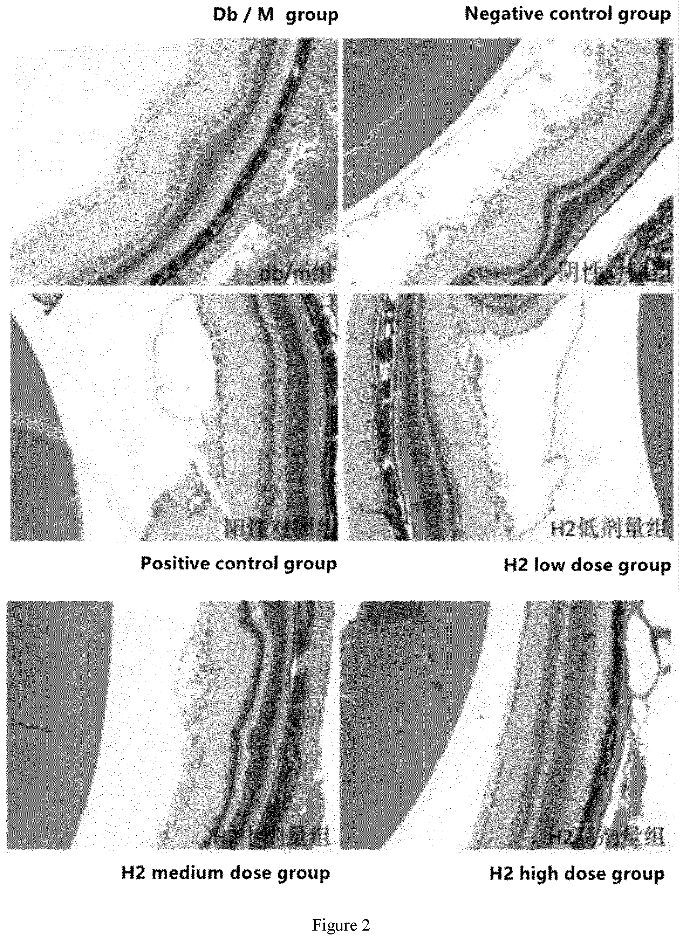

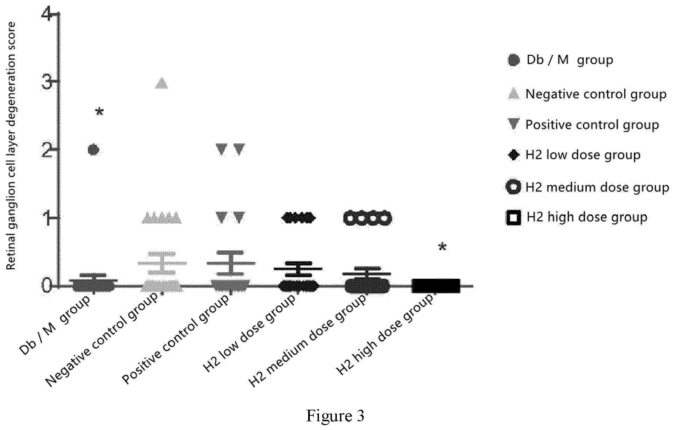

During the test period, compared with the animals in normal control group, the retinal ganglion cell layer of animals in each group had different degrees of degeneration except for the test drug H2 high-dose group. According to the severity of the degeneration of the retinal ganglion cell layers, there were 0 to 4 grades according to the pathological grading principle in the literature (Fermented Milk has Anti-diabetic Effects: Anti-diabetic Effect of Fermented Milk Containing Conjugated Linoleic Acid on Type II Diabetes Mellitus, Korean J. Food Sci. An., Vol. 36, No.2 (2016)). The larger the value, the more severe the disease. The grading scores for degeneration of retinal ganglion cell layer of each group animals were shown in Table 4 and Figures 2 to 3.

**Table 4 Pathological staining results for animal eyeball**

| Animal No. | Left eye | Retinal degeneration, ganglion cell layer | Right eye | Retinal degeneration, ganglion cell layer |
|---|---|---|---|---|
| 1M0101 | N | - | N | - |
| 1M0102 | N | - | N | - |
| 1M0103 | N | - | N | - |
| 1M0104 | N | - | N | - |
| 1M0201 | N | - | N | - |
| 1M0202 | N | - | N | - |
| 1M0203 | N | - | N | - |
| 1M0204 | N | - | N | - |
| 1M0301 | N | - | N | - |
| 1M0302 | E | 2 | N | - |
| 1M0303 | N | - | N | - |
| 1M0304 | N | - | N | - |
| 3M0101 | N | - - | N | - |
| 3M0102 | E | 1 | N | - |
| 3M0103 | E | 1 | N | - |
| 3M0104 | N | - | N | - |
| 3M0201 | E | 3 | N | - |
| 3M0202 | N | - | N | - |
| 3M0203 | E | 1 | N | - |
| 3M0204 | E | - | N | - |
| 3M0301 | E | 1 | N | - |
| 3M0302 | N | - | N | - |
| 3M0303 | N | - | N | - |
| 3M0304 | N | - | E | 1 |
| 4M0101 | / | / | / | / |
| 4M0102 | E | 2 | N | - |
| 4M0103 | N | - | N | - |
| 4M0104 | N | - | N | - |
| 4M0201 | E | 2 | N | - |
| 4M0202 | N | - | N | - |
| 4M0203 | / | / | / | / |
| 4M0204 | N | - | E | 1 |
| 4M0301 | / | / | / | / |
| 4M0302 | E | 1 | N | - |
| 4M0303 | N | - | N | - |
| 4M0304 | N | - | N | - |
| 5M0101 | E | 1 | N | - |
| 5M0102 | N | - | E | 1 |
| 5M0103 | E | 1 | N | - |
| 5M0104 | E | 1 | E | 1 |
| 5M0201 | N | - | N | - |
| 5M0202 | E | 1 | N | - |
| 5M0203 | N | - | N | - |
| 5M0204 | N | - | N | - |
| 5M0301 | N | - | N | - |
| 5M0302 | N | - | N | - |
| 5M0303 | N | - | N | - |
| 5M0304 | N | - | N | - |
| 6M0101 | N | - | N | - |
| 6M0102 | E | 1 | E | 1 |
| 6M0103 | E | 1 | N | - |
| 6M0104 | N | - | N | - |
| 6M0201 | N | - | E | 1 |
| 6M0202 | / | / | / | / |
| 6M0203 | N | - | N | - |
| 6M0204 | N | - | N | - |
| 6M0301 | N | - | N | - |
| 6M0302 | N | - | N | - |
| 6M0303 | N | - | N | - |
| 6M0304 | N | - | N | - |
| 7M0101 | N | - | N | - |
| 7M0102 | N | - | N | - |
| 7M0103 | N | - | N | - |
| 7M0104 | N | - | N | - |
| 7M0201 | N | - | N | - |
| 7M0202 | N | - | N | - |
| 7M0203 | / | / | / | / |
| 7M0204 | N | - | N | - |
| 7M0301 | N | - | N | - |
| 7M0302 | N | - | N | - |
| 7M0303 | / | / | / | / |
| 7M0304 | N | - | N | - |

| | | | | |
|---|---|---|---|---|
| Note: "N" means normal tissue morphology and structure; "E" means changed tissue morphology and structure; "-" means no such pathological change. | | | | |

During the test period, the animals in db/m group (there is one animal with an abnormal retinal ganglion cell layer, possibly resulted from specimen preparation) and the test drug H2 high-dose group had normal morphology of retinal ganglion cell layers, while other groups of db/db mice had different degrees of degeneration for retinal ganglion cell layers; compared with the negative control group, the use of positive drug metformin hardly improved the symptoms of retinopathy, but H2 provided in the present invention had a good effect of improving retinopathy, and as the increase of H2 dose, the effect was significantly augmented. Finally, the high dose of test drug H2 greatly reduced the degeneration of the retinal ganglion cell layers (P < 0.05), indicating that the test drug H2 had a therapeutic effect on the degeneration of retinal ganglion cell layer in animals. After administration for 12 weeks, no obvious abnormal reaction was found in each group of db/db mice, and there was no statistically significant difference in body weight between groups. No obvious abnormality was shown in gross anatomy, indicating that the test drug did not have obvious toxic or side effects on animals.

### 6.3 Prevent the influence on kidney function

### (1) Changes of blood urea nitrogen (UREA) and blood creatinine (CREA) as well as renal index before and after administration

The changes of blood creatinine (CREA) and renal index before and after administration were shown in Table 5.

Before administration, urea nitrogen (UREA) in db/m group was significantly lower than that in db/db group (P < 0.05). Compared with the negative control group, there was no statistical difference in UREA between the positive drug group and each dose group of test drug H2; after administration for 12 weeks, compared with the negative control group, the medium dose of test drug H2 could significantly reduce the level of UREA (P < 0.001), while there was no significant difference in other groups. UREA change rate indicated that compared with the negative control group, the test drug H2 had a tendency to reduce UREA, and the trend in the medium dose group of test drug H2 was more obvious.

There was no significant difference in serum creatinine (CREA) of each group before administration (P ≥ 0.05). After administration for 12 weeks, compared with the negative control group, the positive drug had a tendency to reduce CREA, but there was no statistical difference; the test drug H2 also showed a tendency to reduce CREA, and the medium dose of test drug H2 could significantly reduce the level of CREA (P < 0.05). Compared with the negative control group, the change rate of CREA in the normal control group was significantly lower (P < 0.05); the positive drug and the test drug H2 presented a trend of reducing CREA, and the test drug H2 had a more significant effect, while the low dose of test drug H2 significantly reduced the change rate of CREA.

The kidney index of db/m group was rather significantly higher than that of db/db group (P < 0.001), and there was no significant difference between db/db groups.

**Table 5 Changes of blood creatinine (CREA) and renal index (MEAN±SEM) before and after administration**

| Groups | Dosage | Renal index | Before | After | Change rate of |
|---|---|---|---|---|---|
| | (mg/kg) | (%) | administration CREA (µmol/L) | administration CREA (µmol/L) | CREA |
| db/m group | - | 0.60±0.02 *** | 7.50±3.00 | 5.42±0.74 | 0.52±0.15 * |
| Blank control group | - | 0.38±0.01 | 5.45±1.96 | 7.73±3.78 | 1.01+0.25 |
| Negative control group | - | 0.38±0.02 | 2.92±0.75 | 4.17±0.56 | 1.00±0.12 |
| Positive control group | 350 | 0.43±0.03 | 4.50±1.38 | 2.78±0.88 | 0.83±0.17 |
| H2 low dose group | 40 | 0.36±0.02 | 3.64±1.36 | 3.75±0.65 | 0.63±0.13 * |
| H2 medium dose group | 80 | 0.46±0.02 | 4.44±2.27 | 1.82±0.76 * | 0.50±0.00 |
| H2 high dose group | 160 | 0.39±0.01 | 5.45±1.42 | 2.5±0.83 | 0.71±0.17 |

| | | | | | |
|---|---|---|---|---|---|
| Note: *P<0.05, **P<0.01, ***P<0.001 vs. negative control group. | | | | | |

### (2) Changes in kidney pathology after treatment

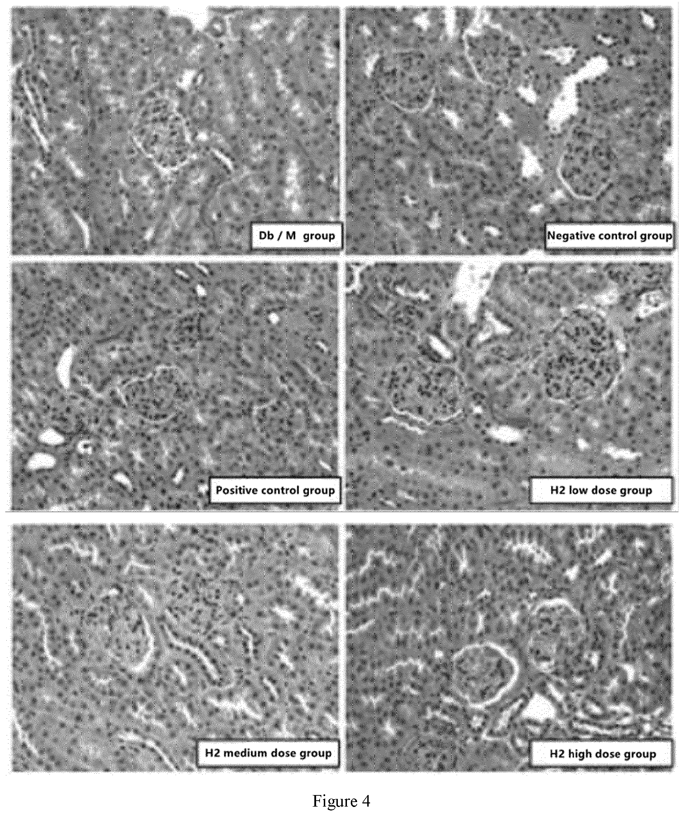

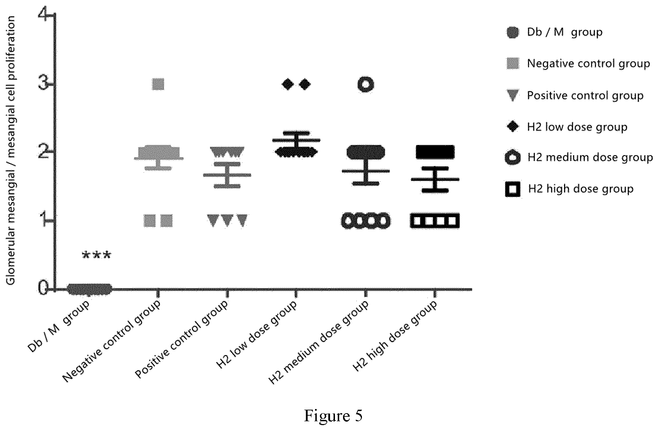

After completion of the test, the kidney was dissected and collected. The histopathological results of the kidney indicated the grades by referring to the principles and standards in literature (Fermented Milk has Anti-diabetic Effects: Anti-diabetic Effect of Fermented Milk Containing Conjugated Linoleic Acid on Type II Diabetes Mellitus, Korean J. Food Sci. An., Vol. 36, No. 2 (2016)). The kidney grading scores for each group of animals were shown in Table 6 and Figure 4. The pathological staining of kidney tissue was shown in Figure 5.

During the test period, compared with db/m, the glomerular mesenteria or mesangial cells of each group of db/db mice all had proliferation (P < 0.001), but the positive drug and the high dose of test drug H2 showed the effect trend of alleviating the proliferation of glomerular mesangum or mesangial cells, but compared with the negative control group, there is no statistical difference.

**Table 6 Kidney grading and scoring results of each group of mice**

| Animal No. | kidney | Basophilic changes in | Monocyte infiltration | Hypertrophy of renal | Proliferation of | Dilation of the | Glomerulosclerosis | Protein casts | Glomerular dilation |
|---|---|---|---|---|---|---|---|---|---|
| | | renal tubules | | tubular epithelial cells | glomerular mesangum or mesangial cells | renal pelvis | | | |
| 1M0101 | N | - | - | - | - | - | - | - | - |
| 1M0102 | E | 1 | 1 | - | - | - | - | - | - |
| 1M0103 | E | - | - | 1 | - | - | - | - | - |
| 1M0104 | N | - | - | - | - | - | - | - | - |
| 1M0201 | N | - | - | - | - | - | - | - | - |
| 1M0202 | N | - | - | - | - | - | - | - | - |
| 1M0203 | N | - | - | - | - | - | - | - | - |
| 1M0204 | N | - | - | - | - | - | - | - | - |
| 1M0301 | N | - | - | - | - | - | - | - | - |
| 1M0302 | N | - | - | - | - | - | - | - | - |
| 1M0303 | N | - | - | - | - | - | - | - | - |
| 1M0304 | E | - | 1 | - | - | - | - | - | - |
| 3M0101 | E | 1- | - | - | 2 | - | - | - | - |
| 3M0102 | E | 1 | - | - | 2 | - | - | - | - |
| 3M0103 | E | - | - | - | 3 | - | - | - | - |
| 3M0104 | E | - | - | - | 1 | - | - | - | - |
| 3M0201 | E | - | - | - | 2 | - | - | - | - |
| 3M0202 | E | - | - | - | 2 | - | - | - | - |
| 3M0203 | E | - | - | - | 2 | 3 | - | - | - |
| 3M0204 | E | - | - | - | 2 | 2 | 1 | - | - |
| 3M0301 | E | - | - | - | 2 | - | - | - | - |
| 3M0302 | N | - | - | - | 1 | - | - | - | - |
| 3M0303 | E | - | - | - | 2 | - | - | - | - |
| 3M0304 | E | - | - | - | 2 | 3 | - | - | - |
| 4M0101 | / | / | / | / | / | / | / | / | / |
| 4M0102 | E | - | - | - | 1 | - | 1 | - | - |
| 4M0103 | E | - | - | - | 2 | - | - | - | - |
| 4M0104 | E | - | - | - | 2 | - | - | - | - |
| 4M0201 | E | - | - | - | 1 | - | - | - | - |
| 4M0202 | E | - | - | - | 2 | - | - | - | - |
| 4M0203 | / | / | / | / | / | / | / | / | / |
| 4M0204 | E | - | - | - | 1 | - | - | - | - |
| 4M0301 | / | / | / | / | / | / | / | / | / |
| 4M0302 | E | 1 | - | - | 2 | - | - | - | - |
| 4M0303 | E | - | - | - | 2 | - | - | - | - |
| 4M0304 | E | - | - | - | 2 | - | - | - | - |
| 5M0101 | E | - | - | - | 2 | - | 1 | - | - |
| 5M0102 | E | 1 | - | - | 2 | - | - | - | - |
| 5M0103 | E | - | - | - | 2 | - | - | - | - |
| 5M0104 | N | - | - | - | 2 | - | - | - | - |
| 5M0201 | E | - | - | - | 2 | - | - | - | - |
| 5M0202 | E | - | - | - | 2 | - | - | - | - |
| 5M0203 | E | - | - | - | 2 | - | - | - | - |
| 5M0204 | N | - | - | - | 2 | - | - | - | - |
| 5M0301 | E | - | - | - | 2 | - | - | - | - |
| 5M0302 | N | - | - | - | 2 | - | - | - | - |
| 5M0303 | E | - | - | - | 3 | - | - | - | - |
| 5M0304 | E | - | - - | - | 3 | - | - | - | - |
| 6M0101 | E | - | - | - | 2 | - | - | - | - |
| 6M0102 | E | - | - | - | 2 | - | - | - | - |
| 6M0103 | E | - | - | - | 2 | - | 1 | - | - |
| 6M0104 | E | - | - | - | 2 | - | - | - | - |
| 6M0201 | E | 1 | - | - | 2 | - | - | 2 | - |
| 6M0202 | / | / | / | / | / | / | / | / | / |
| 6M0203 | E | - | - | - | 1 | - | - | - | - |
| 6M0204 | E | 1 | - | - | 3 | - | - | - | - |
| 6M0301 | E | - | - | - | 1 | - | - | - | - |
| 6M0302 | E | 1 | - | - | 2 | - | - | 1 | - |
| 6M0303 | E | - | - | - | 1 | - | - | - | - |
| 6M0304 | E | - | - | - | 1 | - | - | - | - |
| 7M0101 | E | - | - | - | 1 | - | - | - | - |
| 7M0102 | E | - | - | - | 2 | - | - | - | - |
| 7M0103 | E | - | - | - | 2 | - | - | - | - |
| 7M0104 | E | - | - | - | 2 | - | - | - | - |
| 7M0201 | E | - | - | - | 2 | - | - | - | - |
| 7M0202 | E | - | - | - | 2 | - | - | - | - |
| 7M0203 | / | / | / | / | / | / | / | / | / |
| 7M0204 | E | - | - | - | 1 | - | - | - | - |
| 7M0301 | E | - | - | - | 1 | - | - | - | 1 |
| 7M0302 | E | - | - | - | 1 | - | - | - | - |
| 7M0303 | / | / | / | / | / | / | / | / | / |
| 7M0304 | E | - | - | - | 2 | - | - | - | - |

| | | | | | | | | | |
|---|---|---|---|---|---|---|---|---|---|
| Note: "N" means normal tissue morphology and structure; "E" means changed tissue morphology and structure; "-" means no such pathological change. | | | | | | | | | |

### Example 2

The above-mentioned glycoside compounds could be added to any food, food additive or made into a health product according to a reasonable formula and preparative method, and used to prevent and treat diabetes complications, which belonged to the protection scope of the present application.

The specific examples described above further illustrated the object, technical solutions and beneficial effects of the present invention in detail. It should be construed that above descriptions were only specific examples of the present invention.

## Claims

1. A compound represented by formula (IV), or a pharmaceutically acceptable salt, or solvate thereof for use in the prophylaxis and the treatment of a diabetic complication : 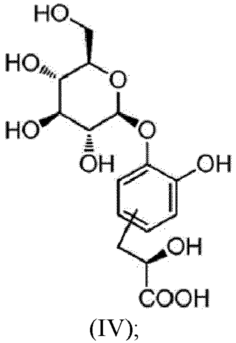 wherein the complication is a microvascular disease.

2. The compound for use according to claim 1, **characterized in that** the complications are those caused by type 1 diabetes.

3. The compound for use according to claim 1, **characterized in that** the complications are those caused by type 2 diabetes.

4. The compound for use according to claim 1, **characterized in that** the microvascular disease is nephropathy and retinopathy.

5. The compound for use according to claim 1, **characterized in that** said drug is administered by sublingual administration, inhalation, oral administration or injection.

## Patentansprüche

1. Verbindung, die durch Formel (IV) wiedergegeben wird, oder ein pharmazeutisch unbedenkliches Salz oder Solvat davon zur Verwendung bei der Prophylaxe und der Behandlung einer diabetischen Komplikation: 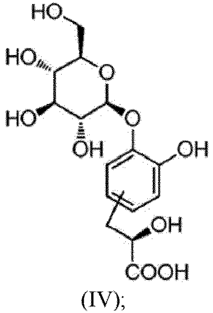 wobei es sich bei der Komplikation um eine mikrovaskuläre Erkrankung handelt.

2. Verbindung zur Verwendung nach Anspruch 1, **dadurch gekennzeichnet, dass** es sich bei den Komplikationen um diejenigen handelt, die durch Typ-1-Diabetes verursacht werden.

3. Verbindung zur Verwendung nach Anspruch 1, **dadurch gekennzeichnet, dass** es sich bei den Komplikationen um diejenigen handelt, die durch Typ-2-Diabetes verursacht werden.

4. Verbindung zur Verwendung nach Anspruch 1, **dadurch gekennzeichnet, dass** es sich bei der mikrovaskulären Erkrankung um Nephropathie und Retinopathie handelt.

5. Verbindung zur Verwendung nach Anspruch 1, **dadurch gekennzeichnet, dass** der Arzneistoff durch sublinguale Verabreichung, Inhalation, orale Verabreichung oder Injektion verabreicht wird.

## Revendications

1. Composé représenté par la formule (IV), ou sel pharmaceutiquement acceptable, ou solvate correspondant pour une utilisation dans la prophylaxie et le traitement d'une complication diabétique : 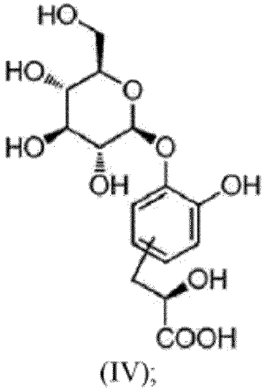 la complication étant une maladie microvasculaire.

2. Composé pour une utilisation selon la revendication 1, **caractérisé en ce que** les complications sont celles causées par un diabète de type 1.

3. Composé pour une utilisation selon la revendication 1, **caractérisé en ce que** les complications sont celles causées par un diabète de type 2.

4. Composé pour une utilisation selon la revendication 1, **caractérisé en ce que** la maladie microvasculaire est une néphropathie et une rétinopathie.

5. Composé pour une utilisation selon la revendication 1, **caractérisé en ce que** ledit médicament est administré par administration sublinguale, inhalation, administration orale ou injection.
